# EUROPEAN PATENT SPECIFICATION

(11) **EP 1 284 669 B1**
(45) Date of publication and mention of the grant of the patent: **06.12.2006**
(21) Application number: 01934276.5
(22) Date of filing: 20.05.2001
(51) Int. Cl.: A61B 18/02, A61F 9/007

(54) **CATARACT SURGERY DEVICES AND METHODS FOR USING SAME**
KATARAKTCHIRURGIEINSTRUMENTE UND METHODEN ZUR VERWENDUNG DERSELBEN
DISPOSITIFS DE CHIRURGIE DE LA CATARACTE ET PROCEDES FAISANT INTERVENIR LEUR UTILISATION

(30) Priority: 22.05.2000 US 205554 P; 25.04.2001 US 286306 P
(43) Date of publication of application: 26.02.2003
(73) Proprietor: ITOS International Ltd, 3rd floor, Jonsim Place 228 Queen's road East Wanchai Hong Kong (HK)
(72) Inventor: BEN-NUN, Joshua, 40291 Post Vitkin (IL)
(74) Representative: Schmitz, Jean-Marie
(86) International application number: PCT/IL2001/000448
(87) International publication number: WO 2001/089401

(56) References cited:
- WO-A-97/41784
- GB-A- 1 473 855
- US-A- 5 254 116

## Description

### FIELD OF THE INVENTION

The present invention relates to the field of devices for eye surgery in general and to the field of devices for cataract surgery in particular.

### BACKGROUND OF THE INVENTION

Surgical removal of cataracts is well known in the art. In cataract surgery, the content of the eye lens is completely removed leaving only the posterior lens capsule, in which an artificial lens may be subsequently installed. It is appreciated that one of the main risks in cataract surgery is a potential damage, e.g. rupture, of the lens capsule. In the past, it was common practice to "freeze" the entire lens using appropriate means and then, to remove the lens in its entirety via a large opening which is formed in the cornea, specifically, along the *Cornea Limbus.* This procedure resulted in damage to the lens capsule and to the vitreous body and is, therefore, no longer in use.

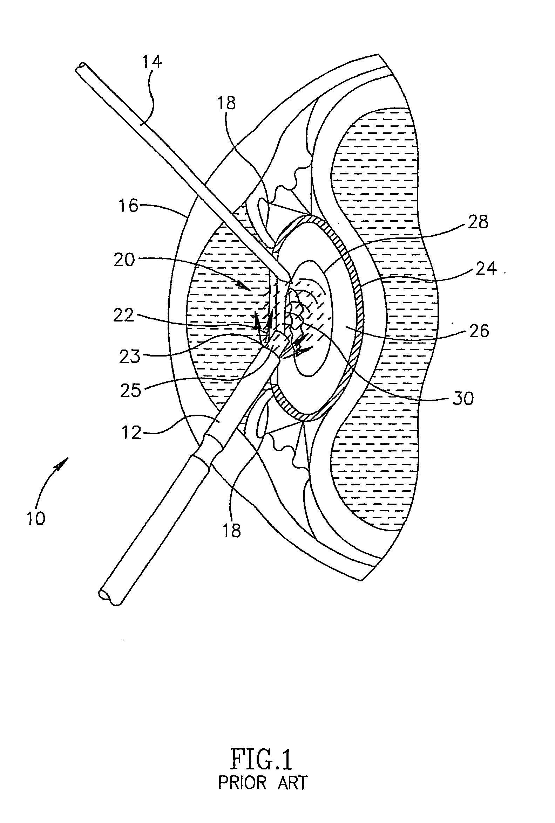

Presently, there are a number of known methods for removing cataracts. Fig. 1 schematically illustrates a cross-sectional view of a human eye 10 during cataract surgery in accordance with one, commonly used, prior art method. A surgical instrument 12 and, optionally, a manipulation device 14, are inserted into eye lens 20 via cornea 16, a preferably dilated pupil 18 and an opening formed in the anterior capsule of lens 20. As is known in the art, lens 20 includes a core 28, known as the *nucleus,* which is formed of a relatively hard tissue. Nucleus or cataract 28 is surrounded by a layer 26 of relatively soft, gel-like tissue, known as the cortex, which fills lens capsule 24.

The soft tissue in cortex layer 26 is typically removed gradually using a vacuum suction device and/or a "scooping" device (not shown in the drawings). To remove nucleus 28, the hard tissue is typically, first, broken into small fragments and/or dissolved using appropriate instruments and/or solutions and is then removed gradually by suction and/or "scooping" as described above. Alternatively, the entire nucleus can be removed in one piece. However, this requires cutting a large opening in the cornea.

Fig. 1 illustrates one method of breaking a nucleus 28 using directional ultrasonic transmission. According to this method, instrument 12 includes a device 25, generally known as a Phacoemulsifier (Hereinafter: "Phaco"), which transmits intense ultrasonic energy into the nucleus 28. The crushing effect of the ultrasonic transmission of Phaco device 25 is typically enhanced by a stream of liquid 22 supplied from an external sleeve 23 of instrument 12, which liquid typically includes a dissolving agent. It is appreciated that, during surgery, a constant supply of liquids is generally required to compensate for escape of intraocular liquids and/or to assist in dissolving the content of lens 20. In the example shown in Fig. 1, the supply of liquid 22 via sleeve 3 is utilized both as a dissolving agent and as a compensatory liquid supply. However, it is appreciated that a separate liquid supply may additionally or alternatively be used.

Manipulation device 14 typically includes a thin, pointed instrument. For example, the thin pointed instrument can be a needle or a spatula, which provides partial counter-support to the operation of instrument 12 on nucleus 28. Such a device enables the surgeon to manipulate nucleus 28 by pushing it to a desired position and to temporarily support the nucleus at the desired position. However, it should be noted that the ability of the surgeon to manipulate and control nucleus 28 using device 14 is limited, due to various physical parameters. For example, the "angle of the attack" of device 14 on the traction between device 14 and the surface of nucleus 28 can be manipulated, using device 14, only by pushing and not by pulling.

Medical follow up studies reveal that the quality of the post-operative optical results depends on the size of the incision made during surgery, where smaller incisions are usually associated with better post-operative results.

An additional development favoring the reduction of the incision size is the availability of foldable artificial lenses, which can be introduced into the eye and inserted into the capsula while folded inside a needle-like device of relatively small diameter.

Unfortunately, ultrasonic systems such as the Phacoemulsifier are relatively expensive. Moreover, during the operation, the surgeon cannot observe a clearly defined border of the crushing action of the Phaco device 25. Thus, the inexperienced surgeon might inadvertently damage the posterior capsule of the lens, resulting in poorer post-operative results.

Additionally, the geometry of the crushing zone around the tip of the Phaco device 25 is not constant, and varies for different sonication intensities, while having no visible cue which the surgeon can use to determine the precise crushing range from the tip of the Phaco device 25.

Consequently, there is a steep learning curve for the surgeon, requiring a relatively long training period and resulting in lower quality of the post-operative results during the training period.

Furthermore, in certain cataract cases, the degree of hardening of the cataract nucleus 28 is such that the Phaco device 25 cannot crush it, thus, requiring the surgeon to broaden the small incision in order to remove the whole cataract nucleus.

WO 97/41784, to the present inventors, describes a cryomanipulator, for use in conjunction with a surgical device. Upon activation of coolant flow to a cryogenic tip, mammalian tissue contacting the tip freezes and adheres to the tip. Repeated activation and deactivation of the freezing activity allows repositioning of the tip, for gripping of various tissue areas and manipulation of these tissues. Fig. 4a of this document shows a surgical apparatus which comprises three tubes: a first one 84 serving as inflow for coolant, a second one 82 extending over the first one 84 and having a rounded tip 72 serving as expansion chamber for the coolant outflow, a third one 52/54 serving as a sleeve and extending over the second one. Upon activation a complete circular freezing zone of tissue is formed, corresponding to the shape and area of the apex of the tip, which contacts the tissue. It has been found by the inventors that drilling through a frozen area in order to emulsify a cataract is most difficult, therefore the cryomanipulator of WO 97/41784 is unsuited for use in a procedure in which drilling through the center of this frozen area is required for removal of a cataract.

### SUMMARY OF THE INVENTION

The present invention seeks to provide an improved device for manipulating tissue during surgery. The manipulation device of the present invention is particularly useful in intraocular surgery, particularly in cataract removal surgery.

The present invention provides a surgical apparatus according to claim 1.

### BRIEF DESCRIPTION OF THE DRAWINGS

The invention will now be described with reference to the accompanying drawing figures, where like reference numerals or characters indicate corresponding or like components. In the drawings:
Fig. 1 is a schematic, side view, cross-sectional illustration of a human eye during cataract surgery in accordance with the prior art;
Fig. 2 is a perspective view of a thermal cutting tool in operation;
Fig. 3 is a perspective view of a thermal probe in operation;
Fig. 4 is a schematic, side view, cross-sectional illustration of a human eye during cataract surgery;
Fig. 5 is a side view of the thermal cutting tool housing;
Fig. 6 is a perspective view of the thermal probe;
Fig. 7A is a cross sectional view of the thermal portion of the thermal probe of Fig. 6;
Fig. 7B is a rear view of the thermal portion of the thermal probe of Fig. 6;
Fig. 7C is cross sectional view of the thermal portion of the thermal probe taken along line 7C-7C of Fig. 6;
Fig. 7D is a cross sectional view of an alternate thermal portion for the thermal probe of Fig. 6;
Fig. 8 is a perspective view of a system and apparatus in accordance with ah embodiment of the invention;
Fig. 9 is a cross sectional view of the tip of the apparatus detailed of Fig. 8;
Fig. 10 is a perspective view of the drill bit in accordance with the embodiment of the invention;
Fig. 11 is a cross sectional view of the handle in accordance with the apparatus detailed in Fig. 8;
Fig. 12 is a cross sectional view of the shaft holding unit in accordance with the embodiment of the invention, with the shaft removed;
Fig. 13 is a rear view of the apparatus detailed in Fig. 8;
Figs. 14-16 are cross sectional diagrams, detailing tube arrangements in the handle, at various rotations, of the embodiment of the invention;
Fig. 17 is a top view of the foot switch of the embodiment of the invention;
Fig. 18 is a cross sectional view of the foot switch of Fig. 17, taken along line 18-18; and
Fig. 19 is perspective view of the apparatus of Fig. 8 in an exemplary operation.

### DETAILED DESCRIPTION OF THE DRAWINGS.

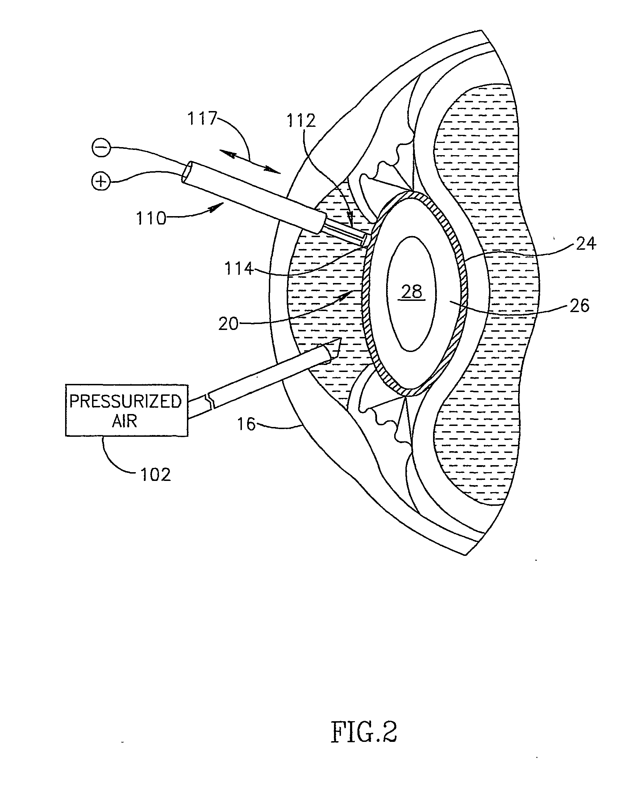

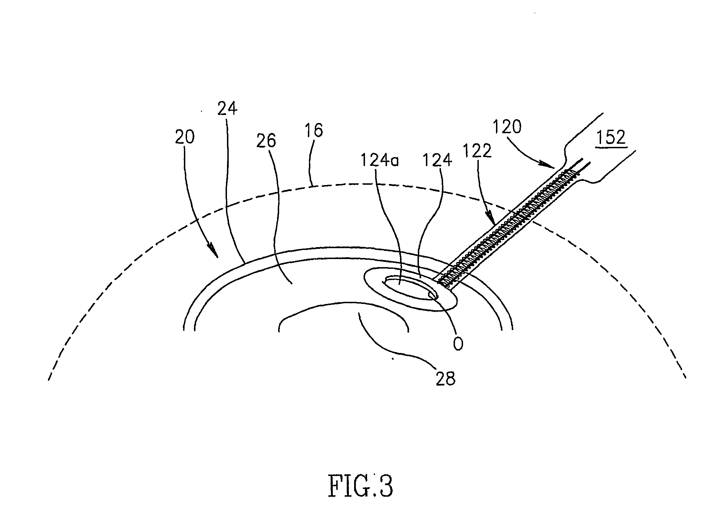

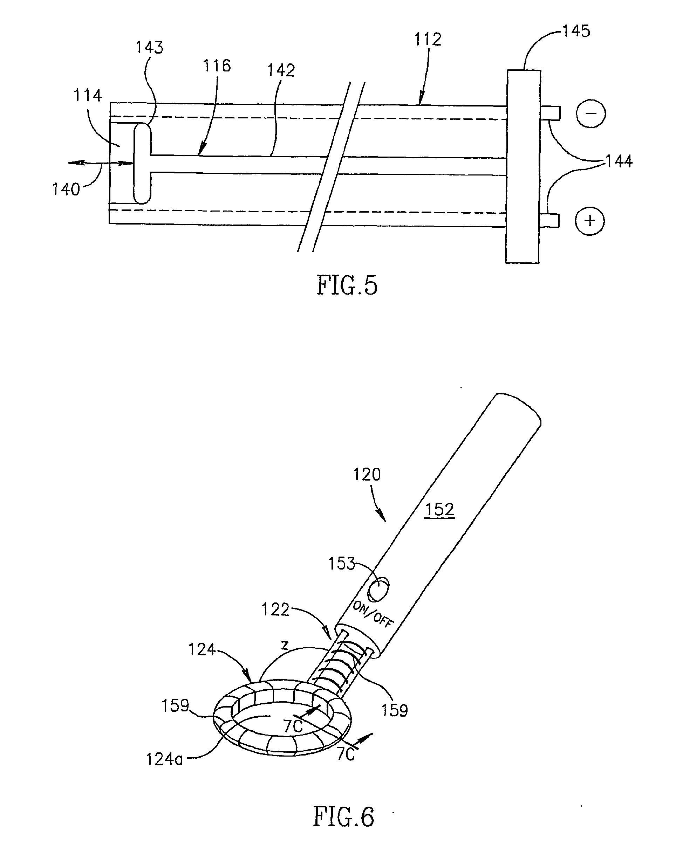

Figs. 2-4 show the instrumentation of the present invention performing cataract removal. Initially, the area surrounding the lens 20 is placed under a pressurized air field from a pressurized air source 102. As shown in Fig. 2, a thermal cutting tool 110 (also shown in Fig. 5 and in additional detail below) is then maneuvered through an incision in the cornea 16, whereby its thermal portion 112, and in particular its beak or tip 114 contacts the lens capsule 24. An opening O (Fig. 3) is created in the lens capsule 24 by an electrical pulse through the tool 110 that creates a pulse of heat at the tip or beak 114. The pulse of heat concentrates at the tip or beak 114, as a result of the configuration of the slots 116 in the thermal portion 112. With the opening O now created in the lens capsule 24, the thermal cutting tool 110 is removed from the site, typically in the direction of the double headed arrow 117 (the insertion direction).

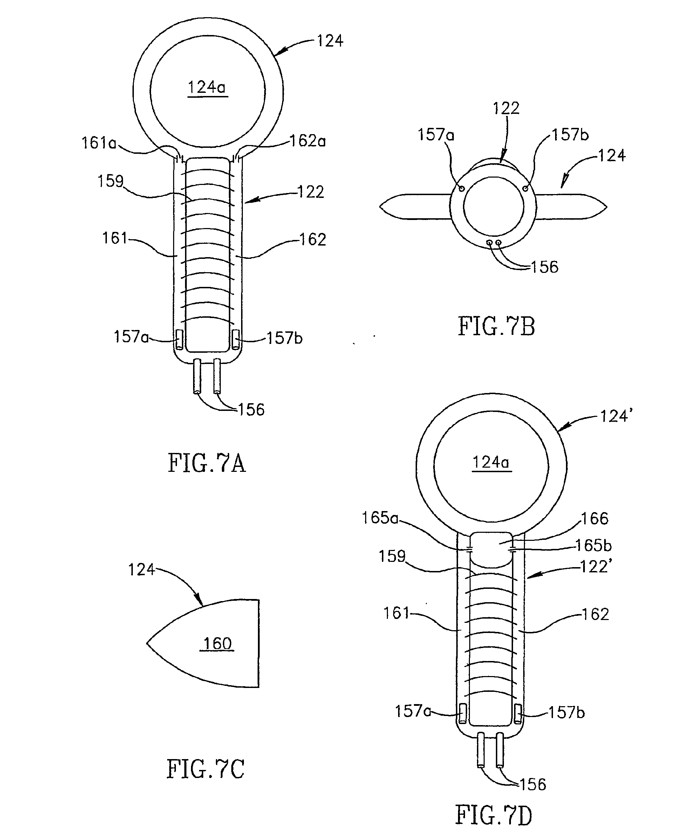

In Fig. 3, with the opening O now created, a thermal probe 120 (also shown in Figs. 6 and 7A-7C and in additional detail below), is then maneuvered through a conventional made incision in the cornea 16. The thermal portion 122 of the thermal probe 120, specifically its torroidal portion 124, contacts the lens capsule 24 over and in alignment with the opening O.

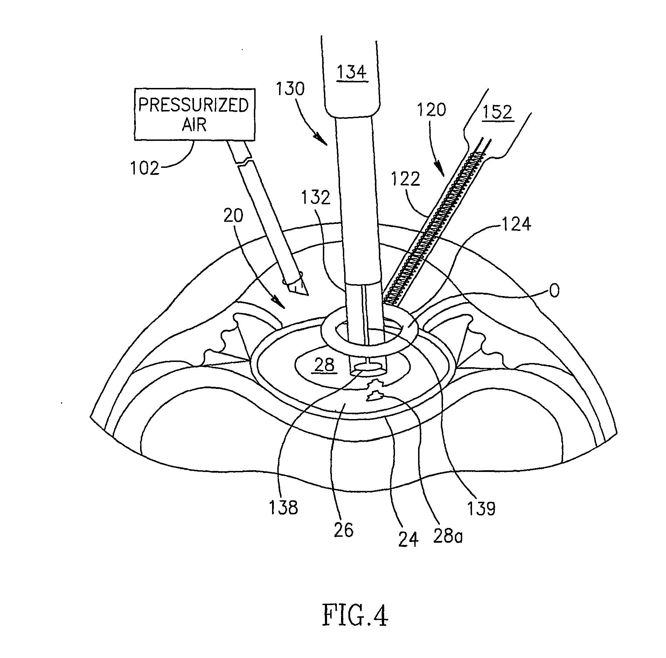

As detailed specifically in Fig. 4, typically through the same corneal opening that was occupied by the thermal cutting tool 110, a drilling unit 130 of a Cataract Removing Device (CRD), the entire CRD detailed in commonly owned US Patent Application Nos. 08/851,505 and 09/156,982, and in particular its housing 132 enters the anterior chamber and the opening O, as the surgeon manipulates its handle 134.

The thermal probe 120 is now activated, specifically such that the torroidal portion 124, having an opening 124a, cools to a temperature where the capsule tissue adheres to the lower edge of the torroidal portion 124 and portions of the subcapsular cortex 26 below the capsule tissue also freeze. This results in the torroidal portion 114, having a firm freeze grip on the capsule 24. This freeze gripping is such that the integrity of this rounded opening is maintained, and specifically that a tight seal is provided to the opening, stabilizing the capsule 24 edges and rendering it free of shearing forces and leakage during the procedure. Typical cooling temperatures for the torroidal portion are approximately -5C to -10C.

The drilling unit 130 can now be activated, as the surgeon, typically with handle controls, causes the drill bit 136 to move axially, such that the drill blade 138 at the end of the drill shaft 139 extend from the housing 132 and into the cortex 26. The drill bit 136 is rotated by motors (not shown) in the drilling unit 130 at speeds as to generate turbulence that decompose and dissolve the cataract (nucleus) 28 into pieces 28a and further to emulsify it into viscous particles within the cortex 26. These speeds may be, for example, approximately 100,000 rpm. During the rotation, irrigation and aspiration, both contemporaneous or non-contemporaneous, may be carried out through the housing 132. This procedure continues until the desired amount of nucleus 28, typically all of it, is emulsified and aspirated.

Upon completion of the procedure, the torroidal portion 124 is heated, typically by ceasing gas flow therethrough, to a temperature where the gripping by freezing is released and the thermal probe 120 and drilling unit 130 can be removed from the surgical site. A lens may then be implanted into the capsule 24 of the lens 20 by conventional procedures.

Fig. 5 details the thermal portion 112 of the cutting tool 110. The thermal portion 112 is typically tubular, cylindrical in shape, although other shapes are also permissible. Slots 116 are oppositely disposed with respect to the axis (indicated by the double headed arrow 140). The slots 116 typically include a channel portion 142 continuous with a head portion 143, so as to be "T" shaped. This "T" shape is such that thermal load at the tip 114 will be maximized upon passage of electricity through electrical connectors 144 at the handle interface 145. The thermal portion 112 is of a conducting material, including metals such as steel, titanium or the like, that coupled with the "T" shaped slots 116 concentrates the heat at the tip or beak 114. The heat is typically received as energy pulses through the electrical connectors 144 at the handle interface 145.

Typically, there are two slots 116, equidistant from each other. One or three or more slots are also permissible, provided they are configured to concentrate the heat at the tip or beak 114.

Figs. 6 and 7A-7C detail the thermal probe 120. This probe 120 includes a thermal portion 122 and terminates in a torroidal portion 124, with an opening 124a therein. A handle 152 attaches to the thermal portion 122 via heating 156 and cooling 157a, 157b connections (or ports) and there is a control system (not shown) along with associated mechanisms for providing heating and cooling to the thermal 122 and torroidal portions 124. There are also controls, activatable by the surgeon on the handle 152, including an on/off switch 158, and temperature controls, for the surgeon to control precise heating and cooling at the torroidal portion 124.

Heating is achieved via conduction (coils 159) that receive electricity from the heating connections 156. Cooling is achieved via gas circulation and expansion in the hollow chamber 160 of the torroidal portion 124. The gas is typically CO₂ (Carbon Dioxide) and N₂O (Nitrous Oxide), released from a gas source (not shown) where these gasses are compressed, that cools upon expansion. Gas enters the thermal portion 122 through connection 157a and travels through a conduit 161 to a bore 161a, where it enters the torroidal portion 124. Gas leaves torroidal portion 124 through an outflow conduit 162, via a bore 162a. The outflow conduit connects to outflow lines (not shown) at connection 157b, where the outflow gas is discharged to the ambient environment.

Heating and cooling operate together, and as a result, cooling is localized at the torroidal portion 124 so at to be confined to the surface of the lens capsule 24, leaving the remaining environment at warmer temperatures, suitable for avoiding instrument and tissue damage from freezing.

The torroidal portion 124 is of a rounded triangular cross section, to enable smooth insertion of the housing 132 of the drilling unit 130 through the requisite opening O in the lens capsule 24. Other shapes are also suitable, provided, they can make a freezing contact with a significant amount of tissue, to properly freeze grip the tissue and maintain the sealing and integrity of the opening O, as detailed above. This the torroidal portion is at an angle "z" of anywhere for 0 to 45 degrees, and is typically approximately 30 degrees, with the torroidal opening 124a dimensioned to accommodate surgical openings, like opening O, that are typically approximately 1.0 to 2.0 mm.

The thermal portion 122 and torroidal portion 124 are typically an integral, one-piece member made of materials such as metals including stainless steel, titanium or the like. The materials may be bendable, such that the surgeon can adjust the thermal probe 120 prior to or during surgery.

Turning to Fig. 7D, there is shown an alternate embodiment of the thermal portion 122'. This thermal portion 122' is similar in all aspects to thermal portion 122, shown in Figs. 7A and 7B and described above, except as described hereinbelow. Specifically, in this thermal portion 122', the torroidal portion 124' is a solid member (otherwise similar in all aspects to torroidal portion 124 as shown and detailed above, except where indicated below), made of a thermal (cool) conducting material such as gold, or other good thermal (cool) conductor. It is joined to the remainder of the thermal portion 122' by conventional metal joining techniques. Between gas supply 161 and gas outflow 162 conduits is a chamber 165 in contact with the torroidal portion 124'. Bores 166a, 166b connect the respective gas conduits 161, 162 with the chamber 165.

The chamber 165 serves as an expansion chamber for the incoming gas, allowing it to cool. This cooling is transferred to the torroidal portion 124', that because of its good conduction, allows for cooling to spread rapidly over the entire torroidal portion 124'. Cooling is thus similar to that described above and this thermal portion 122' operates identically to thermal portion 122.

This surgical procedure and the tools associated therewith allows the capsule to remain intact during the entire surgical procedure.

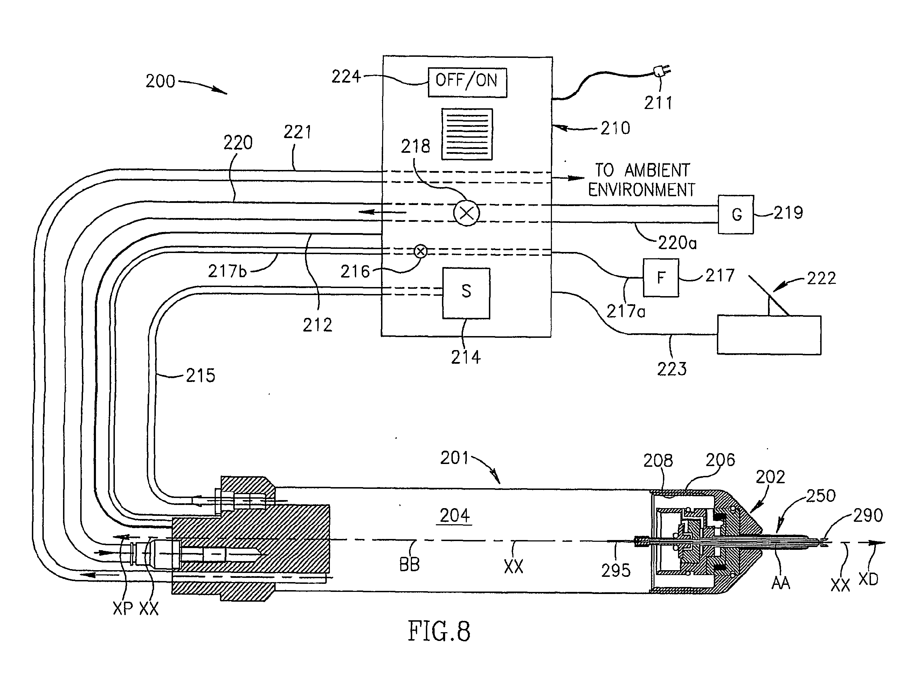

Turning now to Fig. 8, there is shown a system 200 in accordance with a second embodiment of the invention. The system 200 includes an apparatus 201 where the thermal (heating/cooling) and drilling functions, detailed above, have been consolidated in a single instrument.

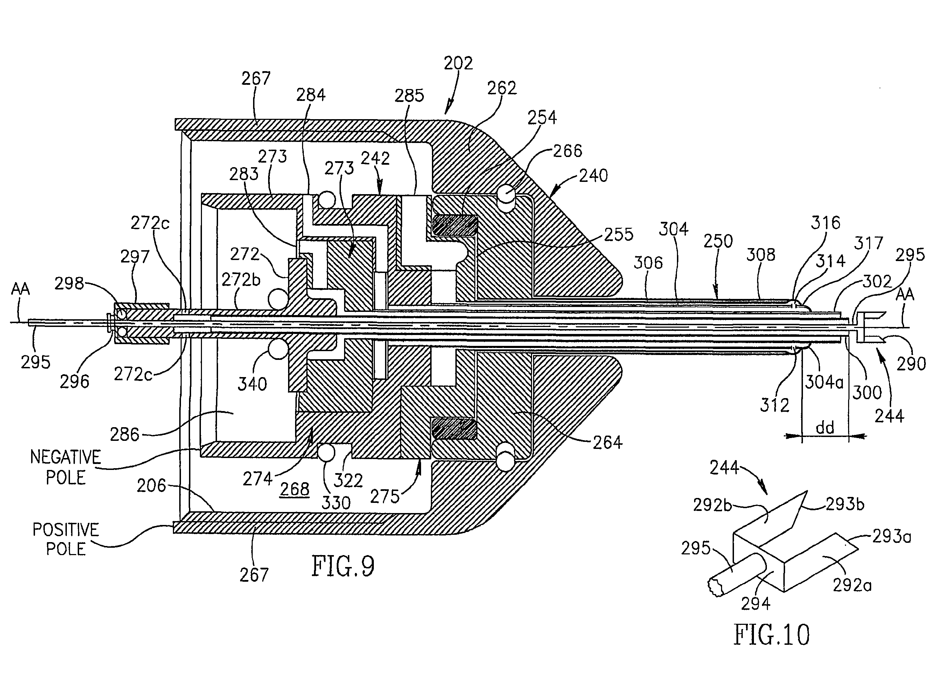

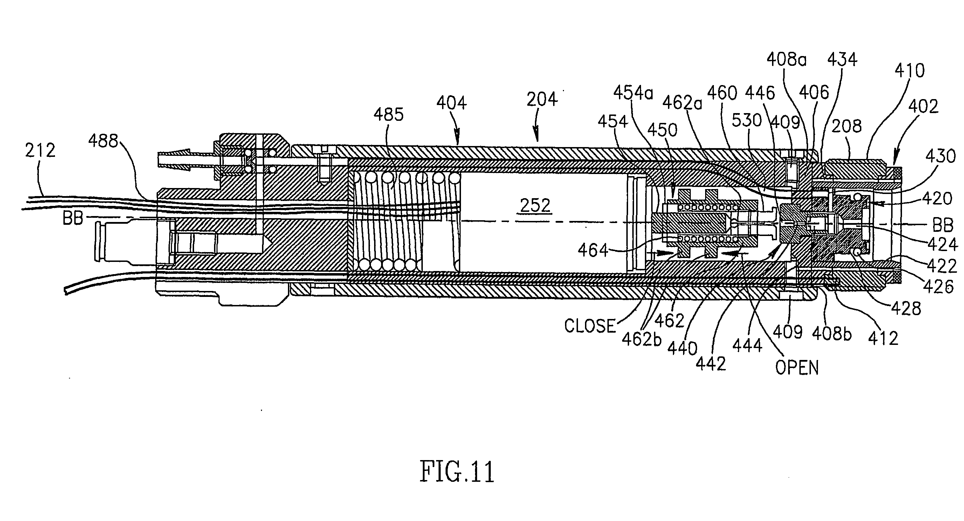

The apparatus 201 includes a tip 202 and a handle 204. The tip 202 is typically disposable and is removable from the handle 204. An axis XX extends through the apparatus 201, and is coaxial with both axis AA (also shown in Fig. 9) extending through the tip 202 and axis BB (also shown in Fig. 11) extending through the handle 204, when the apparatus 201 is properly assembled. For purposes of description and orientation of components for the system 200, and typically the apparatus 201, the terms "proximal" and "distal" will be employed, whereby "distal" is in the direction of the tip 202 and more particularly in the direction indicated by arrow head XD on axis XX, and "proximal" is in the direction of the handle 204 and more particularly in the direction indicated by arrow head XP on axis XX.

The tip 202 engages the handle 204 by threads 206, 208 correspondingly configured on the tip 202 and handle 204 or other similar cooperating arrangement. The apparatus 201 is controlled by a control unit 210, that connects to a power source (not shown), such as an electrical outlet or the like via a plug 211 or the like. The control unit 210 provides power to the handle 204 through electrical lines 212. There is a suction unit (S) 214, inboard the control unit 210, but it could also be outboard of the control unit 210, to provide suction to the handle 204 and tip 202 through suction line 215. The control unit 210 also includes a valve 216 to control the flow of irrigation fluid, from a fluid source (F) 217 (through a line 217a), to the handle 204 and tip 202, through fluid line 217b. There is another valve 218 to control gas flow to the handle 204 and tip 202, from a gas source (G) 219, through a gas line 220 (the gas source 219 connecting to the valve 218 via line 220a). The gas or coolant source 219 is typically of compressed gases, that cool upon expansion, such as, for example, CO₂ (Carbon Dioxide) and N₂O (Nitrous Oxide). There is also a gas outflow line 221, connected to the handle 204, from which gas is discharged to the ambient environment.

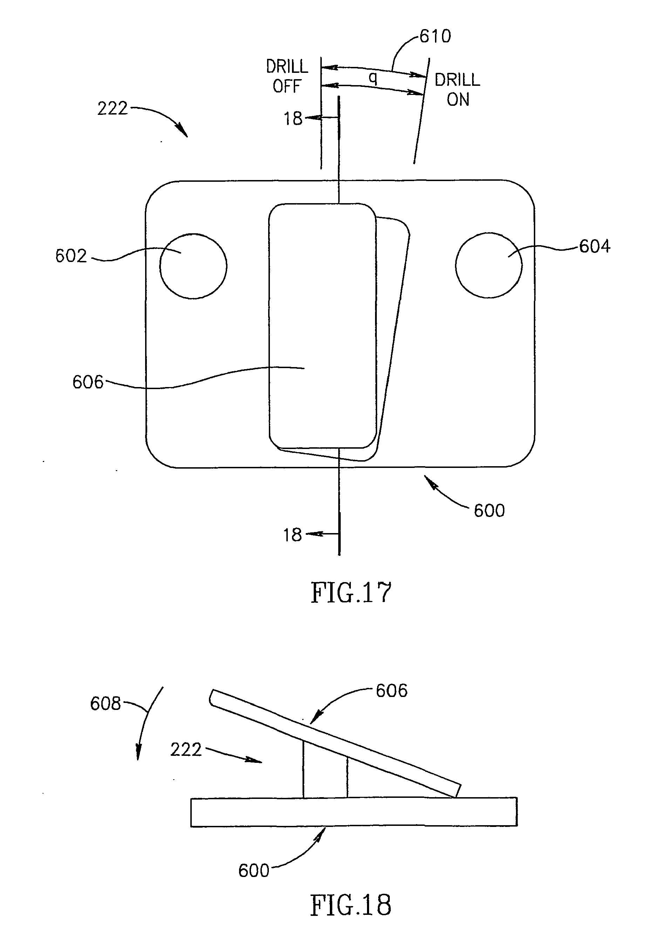

A foot switch 222 (detailed below in Figs. 17 and 18) is electrically connected to the control unit 210 by a line 223, and controls many functions of the above detailed system 200. There is also an ON/OFF switch 224 on the control unit 210 that controls power to the control unit 210 and the foot switch 222.

Turning also to Fig. 9, there is shown the tip 202, that is formed of a head portion 240, and a core 242, and supports a drill member 244, housed in a series of overlapping tubes 250 with spaces therebetween, that are mounted in the head portion 240 and core 242 respectively. The core 242 is separated from the head portion 240 by a ring of electrical insulation 254, and an air gap 255, such that the head portion 240 and core 242 do not contact each other and thus, can maintain different electrical potentials to form an electrical circuit (detailed below).

The head portion 240 is typically formed of two members, an outer member 262 and an inner member 264 with an O-ring 266 therebetween, of materials such as conductive polymers or other similar materials, to provide shock absorbance of from vibrations, due to the high rpm speeds of the drill member 244 (typically approximately 80,000 rpm). The outer member 262 includes an extended portion 267, and a chamber 268 is present between the extended portion 267 that envelopes the core 242. This extended portion 267 typically include threads 206 on its inner side, that correspond with threads 208 on the handle 204, so as to allow joining and retaining of the tip 202 on the handle 204 when use of the apparatus 201 is desired. The head portion 240 is typically formed of surgical grade steel, other biocompatible metal or other biocompatable material, provided these materials are electrical conductors. Plastic with electrically conducting wires therein is also suitable.

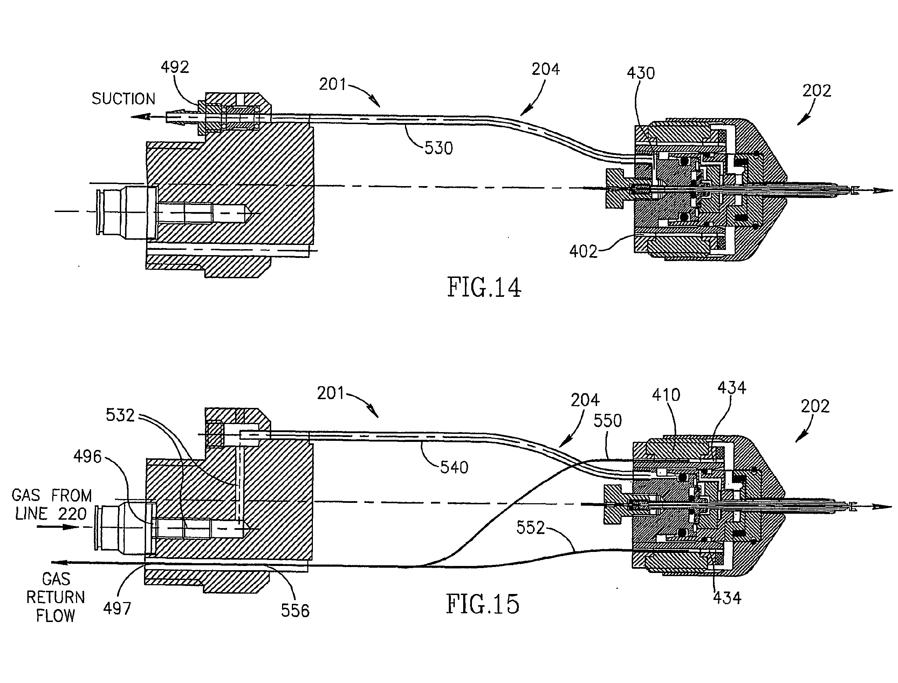

The core 242 is formed from collars 272-275, and supports tubes (detailed below), that protrude from the head portion 240. The collars 272-275 are configured to provide passages 283-285 for suction, fluids and gases into the respective tubes (detailed below), from the respective tubes 530, 540, 550, 552, 560 in the handle 204 (detailed in Figs 14-16, and detailed below). The three inner collars 272-274, form a space 286 dimensioned for receiving a stub 420 on the handle 204.

Turning also to Fig. 10, the drill member 244 is formed of a drill bit 290, with balanced blades 292a, 292b having oppositely tapered ends 293a, 293b, connected to a crossbar 294. While two balanced blades 292a, 292b with oppositely tapered ends 293a, 293b are shown, this is exemplary only, as the drill bit 290 could be modified to support any number of balanced blades, with any end tapering arrangements, provided the drill bit 290 is able to rotate in a balanced manner. Alternately, the drill bit 290 can be in accordance with lens-reducing head, as disclosed (as element 318 and shown in one instance in Fig. 4d) in U.S. Patent No. 5,690,641 (Sorensen, et al.).

The drill bit 290 is mounted on a shaft 295, that extends beyond the head portion 240, that is received by the handle 204. Moving in the proximal direction, prior to the terminal end of the shaft 295, there is a circumferential protrusion 296, that abuts a stop collar 297, that receives the proximal end 272a of the innermost core collar 272, with an O-ring 298 therebetween for shock absorbence and inhibiting friction between the core collar 272 and the stop collar 297.

The drill bit 290 and shaft 295 are made of biocompatible metal such as titanium, stainless steel or the like. The drill bit 290 may be a single piece, or the blades 292a, 292b, and crossbar 294 can be single pieces joined together by conventional metal joining techniques such as welding or the like. The drill bit 290 is joined (at the crossbar 294) to the shaft 295 by conventional metal joining techniques such as welding or the like.

The series of tubes 250, is formed by tubes 300, 302, 304, 306, 308, that are typically concentric and coaxial (with respect to axis AA), so as to properly accommodate the drill member 244 and allow for the respective ingress and egress of fluids, gases and particulates. These tubes 300, 302, 304, 306, 308 are made of surgical grade steel or any other biocompatable metal or other biocompatible material, provided that these materials are electrical conductors.

The innermost or central tube 300 serves as a housing for the drill shaft 295. This tube 300 also provides a passage for suction of fluids, particulates, etc. from the surgical site. It is mounted in the innermost core collar 272. This collar 272 includes a proximal end 272a and a distal end 272b. The proximal end 272a is tubular and is received in the stop collar 297 and abuts the O-ring 298. This proximal end 272a also includes at least one bore 272c (two are shown) through which suction can be brought to the central tube 300.

Moving outward, the second tube 302 is a tube through which fluid is transported to the surgical site. This tube 302 terminates just slightly proximal with respect to the end of the central tube 300. It is mounted in an intermediate core collar 273 in a manner that fluid can pass from the passage 283 in the core collar 273 to the tube 302.

Alternately, the functions of the tubes 300, 302 can be switched. In this case, tube 300 would supply fluid to the surgical site while suction would be provided through the second tube 302. The reminder of the apparatus 201 would be modified accordingly to accommodate this change in tubes 300, 302.

Continuing outward, the third tube 304 and fourth tube 306, when coupled with the requisite spaces/passages/channels/canals in the collars in the tip 202 and in the handle 204, respectively, gas source 219 and gas lines 220, 220a, 221 and controller 210 form a cooling system, that is closed to the ambient environment. Inner tube 304 is a gas transport tube, while outer tube 306 is an outlet tube, through which the gas returns to the apparatus 201. Both tubes 304, 306 are closed to the environment, as the distal end 304a of the inner tube 304 is rounded, so as form a closed space with the second tube 302. This tube 304 includes at least one bore 312, two are shown here, that allow gas to pass from this tube 304 into an expansion chamber 314 of the outlet tube 306.

The expansion chamber 314 receives gas through the bores 312. This expansion chamber 314 is typically formed by an outwardly protruding arced circumferential portion 316, that closes on the inner tube 304, at or near the distal end 304a. This arced portion 316 creates space for expansion of the gas, to create cooling on the outside side of the arced portion 316. These two tubes 304, 306 terminate at approximately the same point, proximal to the second tube 304. The arced circumferential portion 316 coupled with the rounded distal end 304a of the supply tube 304 define a shoulder 317 along the outer side. This configuration confines cooling to the portion of the shoulder 317 formed by at least the arced portion 316 (but typically also includes the other portion of the shoulder 317 formed by the rounded distal end 304a of supply tube 304), so as to have localized cooling. This cooling is localized and at temperatures sufficient to freeze the requisite tissues upon contact with the shoulder 317, and here for example, allowing for gripping or freeze gripping of the capsule 24, without harming any additional tissues. Additionally, the termination of tubes 304 and 306, forming the shoulder 317 is of a larger diameter than inner tubes 300, 302 and coupled with a distance, indicated by dd (typically approximately 0.5 mm), between the shoulder 317 and termination of innermost tube 300, the penetration depth of the drilling unit 244 into the requisite tissue is limited, here for example, the penetration depth into the capsule 24. This allows for maximum safety and effectiveness of the rotating drill blades 292a, 292b.

Tube 304 is mounted in an intermediate core collar 274 in a manner where coolant gas can pass from the passage 284 into the core collar 274 to the tube 304. Similarly, tube 306 is mounted in an outer core collar 275 in a manner where coolant gas can leave the tube 306 by a passage 285 in the collar 275.

The outermost tube 308 is a heater tube. It contacts the expansion tube 306 at its (the heater tube's 308) distal end. Coupled with the expansion tube 306 mounted to a collar 275 of one potential, for example negative, and the outermost tube 308 is mounted on the inner member 264 of the head portion 260 of another potential, for example, typically positive, an electrical circuit is formed. This arrangement is such that when power is received, the outermost or heater tube 308 will become heated. This heating is such that it at least partially confines or localizes the cooling from the gas tubes 304, 306, to the surgical site, at or near the outer side of the expansion chamber 314, while keeping temperatures in the tubes 300, 302, 304, 306 or portions thereof, over which the heater tube 308 extends, within a biocompatible range. This heating limits any backwards conduction of cooling temperatures from the expansion chamber 314, so as to avoid any tissue damage, for example to the cornea 16, iris and other surrounding tissues, from this cooling.

The core 242 and in particular its collars 272-275. are typically formed of surgical grade steel, other biocompatible metal or other biocompatable material, provided these materials are electrical conductors. An O-ring 330 typically surrounds the core 242, at its intermediate collar 274, that includes a circumferential indent 332 to accommodate this O-ring 330. An O-ring 340 also surrounds the innermost core collar 272 at its distal end 272b. These O-rings 330, 340 are made of elastomers or any other materials suitable for sealing and shock absorbence and friction reduction, from correspondingly configured components on the handle 204 (detailed below).

Figs. 11-16 show the handle 204 in detail. Turning to Fig. 11, this handle 204 is formed by a head portion 402, a body 404 and a front plate 406. An axis BB extends through this head portion 402 and body 404, such that it is in alignment with Axis AA of the tip 202, when the tip 202 and handle are joined together. The head portion 402 connects to the body 404 by being screwed into the body 404, via corresponding threads 408a, 408b on the head portion 402 and body 404 respectively, and/or is attached to the front plate 406, by conventional attachment techniques and mechanisms. The handle also includes screws 409 that hold components in place.

The head portion 402 includes an outer ring 410, typically having threads 208 on its outer side, or other locking mechanism, corresponding with the threads 206 or other locking mechanism on the tip 202 at the extended portions 267. This outer ring is typically made of surgical grade steel or other biocompatible material, that is an electrical conductor, for in accordance with the head portion 240 of the tip 202, this outer ring 410 also has a positive potential, to form a portion of the electrical circuit, as detailed above. An electrical supply line 412 contacts the outer ring 410, supplying it with electricity to provide the electrical potential, here for example, positive potential.

The head portion 402 also includes a stub 420 with a circumferential cylinder 422 around it, for engaging the collars 272-275. The stub 420, fits within the space 286 formed by the inner collar members 272-274. The stub 420 includes a central bore 424 for receiving the drill shaft 295 and stop collar 297, along with an O-ring 426 (in accordance with those O-rings detailed above) in a circumferential indent 428, for shock absorbence and friction reduction when the tip 202 and handle 204 are joined together. A passage 430 that receives a tube 530, that extends through the handle 204, which supplies suction is also in the stub 420 and extends to the central bore 424.

The cylinder 422 is dimensioned to extend into the chamber 268, as far as but not covering gas outflow passage 285 in core collar 275. Accordingly, the space 434 between the cylinder 422 and the outer ring 410 provides a portion of the passage for gas return.

The stub 420 and front plate 406 couple to hold a retainer 440. The retainer 440 includes a bore 442 extending axially therethrough (along axis BB), with this bore including a distal chamber 444 and a tail portion 446. The distal chamber 444 is correspondingly configured to seat the stop collar 297. This distal chamber 444 is slightly larger in dimensions than the stop collar 297, to allow its rotation therein and includes an O-ring 248 (in accordance with the O-rings detailed above) at its proximal end for shock absorbenace and friction reduction. The tail portion 446 is of a diameter larger than that of the shaft 295, but of a diameter sufficient to allow shaft 295 rotation in a balanced manner.

A shaft holding unit 450 is connected to a motor 452 by a drive rod 454. This shaft holding unit 450 receives and holds the remaining end of the shaft 295 (that portion proximal to the stop collar 297) so as to be rotated by the motor 452. The shaft holding unit 450 includes a clamping member 460, that is opened to receive the shaft 295 and closed to hold the shaft 295 for rotation, by an inner sleeve 462 that moves between distal and proximal positions to close and open the clamping member 460. A spring 464 that journals the drive rod 454 is typically biased so as to push the inner sleeve 462 distally, whereby the clamping member is in the closed position, holding the shaft 295 with sufficient retention to for rotation. The spring 464 is bounded by a proximal collar 454a of the drive rod 454 and an inwardly extending shoulder portion 462a on the inner sleeve 462.

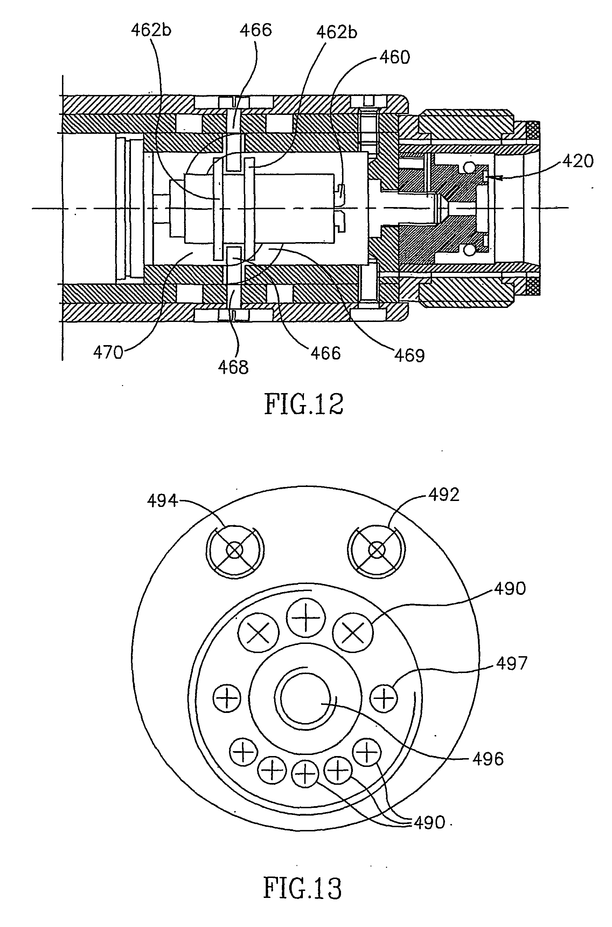

Turning also to Fig. 12, circumferential rings 462b protrude from the inner sleeve 462 and abut teeth 466 of an outer sleeve 468 that travel in a fixed path, formed by grooves 469 in an intermediate sleeve 470. The outer sleeve 468 is moved, typically twisted, such that teeth 466 abut rings 462b, moving them. This moves the inner sleeve 462, ultimately opening and closing the clamping member 460 (around the shaft 295 in an engagement sufficiently tight for rotation) when desired.

The motor 452 is maintained in place by a spring 485. The motor 452 is typically a motor, capable of rotating the shaft at speeds of up to approximately 100,000 rpm, but here, for example, in this apparatus 201 motor speeds of approximately 80,000 rpm are typical. The motor 452 receives power from electrical lines 212, that extend into a canal 488 in the handle 204.

Turning now to Figs. 13-16, and in particular Fig. 13, there is shown the rear or back of the handle 204. The handle 204 includes ports for various lines for electricity 490, suction 492, fluid ingress 494, gas inflow 496 and outflow 497.

Fig. 14 shows the suction tube 530 in the handle 204. The suction tube 530 extends form the port 492 (where it receives suction from line 215 in Fig. 8) to the passageway 430 in the head portion 402 of the handle 204.

Fig. 15 shows gas inflow and outflow in the handle 204. Gas enters through port 496 in the handle 204, through gas line 220. This port 496 opens into a canal 532 in the handle 204, and then to a tube 540, through which gas enters the passage 284 (Fig. 9) in the core collar 274 (Fig. 9) prior to its entering gas supply tube 304 (Fig. 9). Gas outflow is through the passage 285 (Fig. 9) and into the space 434 between the cylinder 422 and outer ring 410 of the head portion 402 of the handle 204. From this space 434, gas flows through tubes 550, 552 (shown in broken lines) in the handle 204 to a canal 556 in the handle 204 and out through ports 497 to gas outflow lines 221 (Fig. 8).

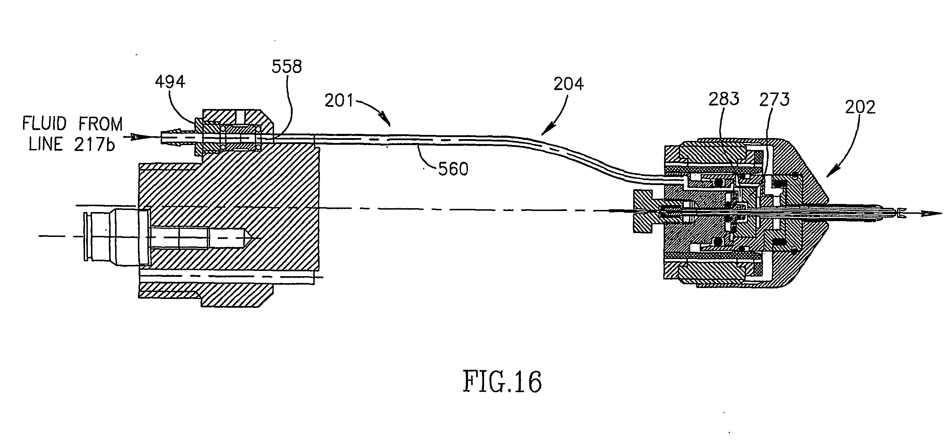

Fig. 16 shows fluid inflow in the handle 204. Fluid enters (from fluid line 217b, shown in Fig. 8) through port 494. This port 494 opens into a canal 558 in the handle 204, and then to a tube 560, through which fluid enters the passage 283 in the core collar 273 prior to its entering fluid supply tube 302 (Fig. 9).

Figs. 17 and 18 detail the foot switch 222. The foot switch 222 includes a baseplate 600, with buttons for activating/ceasing coolant gas 602 and activating/ceasing fluid flow 604. These buttons 602, 604 are activated/deactivated by the surgeon stepping on them. There is also a pedal 606, inclined, which the surgeon steps on to control suction. Suction ranges along a gradient, depending on how far down the pedal 606 is pressed (in the direction of arrow 608)- from no suction, when the pedal 606 is not depressed, to full suction when the pedal 606 is fully depressed. The pedal 606 can also be moved laterally, in accordance with double headed arrow 610, where drilling in OFF, when the pedal is perpendicular to the horizontal, or ON, when the pedal 606 has been moved angularly, corresponding to angle "q" with respect to the vertical.

In operation, the system 200, initially utilizes the procedure similar to that shown in Fig. 2 and described above. As a result of this initial procedure, an opening O', similar to opening O has been created.

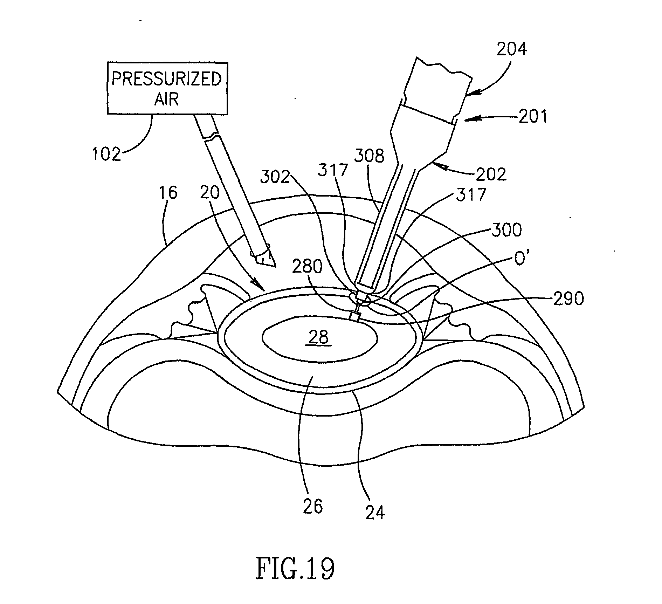

Turning now to Fig. 19, with opening O' now created, the thermal cutting tool (110 Fig. 2) is removed, from the site, as shown and described in Fig. 2. Typically through the same corneal incision (through which the thermal cutting tool was inserted and removed), the apparatus 201, in particular the tip 202 and more particularly, the overlapping tubes 250 are inserted into the corneal incision. Insertion continues into the lens 20, until the surgeon sees abutment of the shoulders 317 against the capsule 24. The drill bit 290, along with portions of central tube 300, having suction, and inner tube 302, with irrigation fluid, have entered the capsule 24 through opening O', and are in the cortex 26, while the shoulders 317 surround the opening O'.

The apparatus 201 is now activated, whereby the gas expands in the expansion chambers 314 to cool the shoulders 317 to a temperature, for example, approximately -5C to -10C, whereby the shoulders 317 have transmitted cooling to the capsule 24, sufficient to freeze it, the freezing adhering at least portions of the shoulders 317 to the capsule 24, so the apparatus 201 has a controlling grip or freeze grip on the capsule 24. This freeze grip is such that the integrity of this rounded opening O' is maintained, and specifically that a tight seal is provided to the opening, stabilizing the capsule 24 edges and rendering it free of shearing forces and leakage during the procedure. Cooling is kept localized to the area of the capsule 24 contacting the shoulders, as the heater tube 308, heats to temperatures of approximately 25C to 35C to confine (localize) this cooling and keep the remainder of the surgical site and tip 202 at biocompatible ranges.

Drilling can now be activated, as the drill bit 290 rotates at speeds as to generate turbulence that decompose and dissolve the cataract (nucleus) 28 into pieces (identical to pieces 28a in Fig. 4) and further to emulsify it into viscous particles within the cortex 26 (as shown and described for Fig. 4 above). These speeds may be, for example, approximately 80,000 rpm. During the rotation, irrigation and aspiration, both contemporaneous or non-contemporaneous, may be carried out through the tubes 300, 302. This procedure continues until the desired amount of nucleus 28, typically all of it, is emulsified and aspirated, through suction tube 300.

Upon completion of the procedure, the gas flow is ceased, allowing the gripping by freezing of the shoulder 317 to be released. Once release is complete, the tip 202 (and thus the apparatus 201) can be removed from the surgical site. A lens may then be implanted into the capsule 24 by conventional procedures.

While the above described systems, apparatus and methods have been shown and described for cataract surgery, this is exemplary only. This is because without or with minimal modifications, these above described systems, apparatus and methods can be used for any kind pathology that involve encapsulated material to be removed while keeping the capsule intact at least during the procedure, to avoid spreading of the intracapsular content outside the capsule (into the surrounding tissue or blood stream). Such pathologies may be for example, encapsulated tumors, meniscus, encapsulated parasites, etc.

While preferred embodiments of the present invention have been described, so as to enable one of skill in the art to practice the present invention, the preceding description is intended to be exemplary only. It should not be used to limit the scope of the invention, which should be determined by reference to the following claims.

## Claims

1. A surgical apparatus (201) comprising:
a first tube (300) including a proximal end and a distal end, said first tube (300) serving as a housing for a drill shaft (295);
a passageway for the inflow and outflow of coolant comprising:
a second tube (304) for communicating with a coolant source (219) extending over at least a portion
of said first tube, said second tube (304) including a proximal end and a distal end, and providing for coolant inflow in said passageway; and
a third tube (306) extending over at least a portion of said second tube (304) and including a proximal and a distal end, said distal end including at least one outwardly extending portion forming an expansion chamber (314) for said coolant inside said third tube (306) and for transferring cooling
from said expansion chamber (314) to outside of said third tube (306), and providing coolant outflow in said passageway;
said second tube (304) including a bore (312) at said distal end for coolant flow into said expansion chamber (314);
a control unit (210) controlling inflow and outflow of said coolant in said second and third tubes (304,306);
a fourth tube (308) extending over at least a portion of said third tube (306), said fourth tube (308) including a proximal end and a distal end and configured for communication with an electrical power source so as to heat when power from said power source is supplied to said fourth tube (308), said fourth tube (308) positioned to extend over at least a portion of said third tube (306) to limit conduction of said cooling toward at least said proximal end of said third tube (306);
said distal end of said first tube (300) extending beyond said distal ends of said second (304) and third tubes (306); and
said distal ends of said second and third tubes (304), (306) extending beyond said distal end of said fourth tube (308).

2. The apparatus of claim 1, additionally comprising a drill member (244), said drill member (244) including a shaft (295) having a distal end and a drill bit (290) at said distal end, said drill member (244) housed at least in partially in said first tube (300).

3. The apparatus of claim 2, additionally comprising a motor (452), said motor (452) coupled with said drill member (244).

4. The apparatus of claim 1, additionally comprising a carrier tube (302) intermediate said first tube (300) and said second tube (304), said carrier tube (302) including a proximal end and a distal end, said distal end extending to a point intermediate said distal ends of said second and third tubes (304), (306) and said distal end of said third tube (306).

5. The apparatus of claim 4, wherein said carrier tube (302) is configured for transport of irrigation fluid from an irrigation fluid source (217).

6. The apparatus of claim 1, wherein said second and third tubes (304), (306) are closed at said respective distal ends, closing said passageway to the ambient environment.

7. The apparatus of claim 6, wherein said closed distal ends of said second tube and said third tube (304), (306) define a shoulder (317), extending outward from said carrier tube (302).

8. The apparatus of claim 7, wherein said distal end of said first tube (300) extends a distance beyond said shoulder (317), said distance corresponding to a maximum penetration depth for said first tube (300).

9. The apparatus of claim 1, wherein said first tube (300) is configured for accommodating suction from a suction unit (214) in communication with said first tube (300).

## Patentansprüche

1. Chirurgische Vorrichtung (201), die Folgendes umfasst:
ein erstes Rohr (300), das ein proximales Ende und ein distales Ende aufweist, wobei das erste Rohr (300) als ein Gehäuse für einen Bohrerschaft (295) dient;
einen Durchgang für den Zustrom und den Ausstrom von Kühlmittel, der Folgendes umfasst:
ein zweites Rohr (304) zum Kommunizieren mit einer Kühlmittelquelle (219), das über mindestens einen Abschnitt des ersten Rohres verläuft, wobei das zweite Rohr (304) ein proximales und ein distales Ende aufweist und für den Einstrom von Kühlmittel in den Durchgang sorgt, und
ein drittes Rohr (306), das über mindestens einen Abschnitt des zweiten Rohres (304) verläuft und ein proximales und ein distales Ende aufweist, umfassend mindestens einen nach außen verlaufenden Abschnitt, der eine Expansionskammer (314) für das Kühlmittel in dem dritten Rohr (306) und zum Übertragen von Kühlmittel aus der Expansionskammer (314) zur Außenseite des dritten Rohres (306) bildet, und der für den Ausstrom von Kühlmittel in dem Durchgang sorgt;
wobei das zweite Rohr (304) an dem distalen Ende eine Bohrung (312) für den Kühlmittelfluss in die Expansionskammer (314) aufweist;
eine Steuereinheit (210), welche den Zustrom und den Ausstrom des Kühlmittels in das zweite und dritte Rohr (304, 306) steuert;
ein viertes Rohr (308), welches über mindestens einen Abschnitt des dritten Rohres (306) verläuft, wobei das vierte Rohr (308) ein proximales und ein distales Ende umfasst und für die Kommunikation mit einer elektrischen Stromquelle konfiguriert ist, um zu heizen, wenn das vierte Rohr (308) mit Strom von der Stromquelle versorgt wird, wobei das vierte Rohr (308) positioniert ist, um über mindestens einen Abschnitt des dritten Rohres (306) zu verlaufen, um die Leitung des Kühlmittels zu dem mindestens proximalen Ende des dritten Rohres (306) zu begrenzen;
wobei das distale Ende des ersten Rohres (300) bis über die distalen Enden des zweiten (304) und dritten (306) Rohres hinaus verläuft; und
wobei die distalen Enden des zweiten und dritten Rohres (304), (306) über das distale Ende des vierten Rohres (308) hinaus verlaufen.

2. Vorrichtung nach Anspruch 1, die des Weiteren ein Bohrerelement (244) umfasst, wobei das Bohrelement (244) einen Schaft (295) mit einem distalen Ende und einen Bohrmeißel (290) an dem distalen Ende aufweist,
wobei das Bohrerelement (244) sich mindestens teilweise in dem ersten Rohr (300) befindet.

3. Vorrichtung nach Anspruch 2, die des Weiteren einen Motor (452) umfasst, wobei der Motor (452) mit dem Bohrerelement (244) gekoppelt ist.

4. Vorrichtung nach Anspruch 1, die des Weiteren ein Trägerrohr (302) zwischen dem ersten Rohr (300) und dem zweiten Rohr (304) umfasst, wobei das Trägerrohr (302) ein proximales und ein distales Ende aufweist,
wobei das distale Ende zwischen den distalen Enden des zweiten und dritten Rohres (304), (306) und dem distalen Ende des dritten Rohres (306) verläuft.

5. Vorrichtung nach Anspruch 4, wobei das Trägerrohr (302) für den Transport von Spülflüssigkeit von einer Spülflüssigkeitsquelle (217) konfiguriert ist.

6. Vorrichtung nach Anspruch 1, wobei das zweite und dritte Rohr (304), (306) an den jeweiligen distalen Enden geschlossen sind, wodurch der Durchgang gegenüber der Umgebung geschlossen wird.

7. Vorrichtung nach Anspruch 6, wobei die geschlossenen distalen Enden des zweiten Rohres und des dritten Rohres (304), (306) eine Schulter (317) definieren, welche von dem Trägerrohr (302) nach außen verläuft.

8. Vorrichtung nach Anspruch 7, wobei das distale Ende des ersten Rohres (300) eine Strecke über die Schulter (317) hinaus verläuft, wobei die Strecke einer maximalen Penetrationstiefe für das erste Rohr (300) entspricht.

9. Vorrichtung nach Anspruch 1, wobei das erste Rohr (300) für das Zulassen eines Saugvorgangs von einer Saugeinheit (214) konfiguriert ist, die in Verbindung mit dem ersten Rohr (300) steht.

## Revendications

1. Appareil chirurgical (201) comportant :
un premier tube (300) comprenant une extrémité proximale et une extrémité distale, ledit premier tube (300) servant de boîtier pour une tige de perçage (295) ;
une voie de passage pour l'afflux et le reflux de réfrigérant, comprenant :
un second tube (304) permettant de communiquer avec une source de réfrigérant (219) s'étendant sur au moins une section dudit premier tube, ledit second tube (304) comprenant une extrémité proximale et une extrémité distale et assurant l'afflux de réfrigérant dans ladite voie de passage ; et
un troisième tube (306) s'étendant sur au moins une section dudit second tube (304) et comprenant une extrémité proximale et une extrémité distale, ladite extrémité distale comprenant au moins une section s'étendant vers l'extérieur en formant une chambre d'expansion (314) pour ledit réfrigérant à l'intérieur dudit troisième tube (306) et pour transférer le refroidissement de ladite chambre d'expansion (314) vers l'extérieur dudit troisième tube (306) et
assurant le reflux de réfrigérant dans ladite voie de passage ;
ledit second tube (304) comportant un alésage (312) à ladite extrémité distale pour l'afflux de réfrigérant dans ladite chambre d'expansion (314) ;
une unité de contrôle (210) contrôlant l'afflux et le reflux dudit réfrigérant dans lesdits second et troisième tubes (304, 306) ;
un quatrième tube (308) s'étendant sur au moins une section dudit troisième tube (306), ledit quatrième tube (308) comprenant une extrémité proximale et une extrémité distale et étant conçu pour communiquer avec une source de courant électrique de manière à chauffer lorsque le courant issu de ladite source de courant est fourni audit quatrième tube (308), ledit quatrième tube (308) étant positionné de manière à s'étendre sur au moins une section dudit troisième tube (306) afin de limiter la conduction dudit réfrigérant vers au moins ladite extrémité proximale dudit troisième tube (306) ;
ladite extrémité distale dudit premier tube (300) s'étendant au delà desdites extrémités distales desdits second (304) et troisième tubes (306) ; et
lesdites extrémités distales desdits second et troisième tubes (304), (306) s'étendant au delà de ladite extrémité distale dudit quatrième tube (308).

2. Appareil selon la revendication 1, comprenant en outre un élément de perçage (244), ledit élément de perçage (244) comportant une tige (295) comprenant une extrémité distale et un trépan (290) à ladite extrémité distale, ledit élément de perçage (244) étant logé au moins partiellement dans ledit premier tube (300) .

3. Appareil selon la revendication 1, comprenant en outre un moteur (452), ledit moteur (452) étant couplé audit élément de perçage (244).

4. Appareil selon la revendication 1, comprenant en outre un tube porteur (302) entre ledit premier tube (300) et ledit second tube (304), ledit tube porteur (302) comprenant une extrémité proximale et une extrémité distale, ladite extrémité distale s'étendant jusqu'à un point entre lesdites extrémités distales desdits second et troisième tubes (304), (306) et ladite extrémité distale dudit troisième tube (306).

5. Appareil selon la revendication 4, dans lequel ledit tube porteur (302) est conçu pour transporter du fluide d'irrigation provenant d'une source de fluide d'irrigation (217).

6. Appareil selon la revendication 1, dans lequel lesdits second et troisième tubes (304), (306) sont fermés auxdits extrémités distales, fermant ainsi ladite voie de passage par rapport à l'environnement ambiant.

7. Appareil selon la revendication 6, dans lequel lesdites extrémités distales fermées dudit second tube et dudit troisième tube (304), (306) délimitent un épaulement (317) s'étendant vers l'extérieur dudit tube porteur (302).

8. Appareil selon la revendication 7, dans lequel ladite extrémité distale dudit premier tube (300) s'étend sur une distance allant au-delà dudit épaulement (317), ladite distance correspondant à une profondeur de pénétration maximale pour ledit premier tube (300).

9. Appareil selon la revendication 1, dans lequel ledit premier tube (300) est conçu pour recevoir de la succion issue d'une unité de succion (214) en communication avec ledit premier tube (300).
